# EUROPEAN PATENT SPECIFICATION

(11) **EP 2 053 918 B1**
(45) Date of publication and mention of the grant of the patent: **23.07.2014**
(21) Application number: 07814154.6
(22) Date of filing: 16.08.2007
(51) Int. Cl.: A61K 31/4745, A61K 47/12, A61K 9/00

(54) **PROPYL PHENOXY ETHERS FOR USE AS DELIVERY AGENTS**
PROPYL-PHENOXY-ETHERN ZUR VERWENDUNG ALS VERABREICHUNGSMITTEL
ÉTHERS PROPYLPHÉNOXY POUR LEUR UTILISATION COMME AGENTS D'ADMINISTRATION

(30) Priority: 18.08.2006 US 838637 P
(43) Date of publication of application: 06.05.2009
(73) Proprietor: Emisphere Technologies, Inc., Cedar Knolls, New Jersey 07927 (US); Song, Jianfeng, Tarrytown NY 10591 (US)
(72) Inventor: SONG, Jianfeng, Tarrytown NY 10591 (US)
(74) Representative: Grünecker, Kinkeldey, Stockmair & Schwanhäusser
(86) International application number: PCT/US2007/076075
(87) International publication number: WO 2008/022242

(56) References cited:
- WO-A1-01/51454
- DE-A1- 3 416 857
- US-A- 2 449 991
- US-A- 4 108 872

## Description

### BACKGROUND OF THE INVENTION

Conventional means for delivering active agents are often severely limited by biological, chemical, and physical barriers. Typically, these barriers are imposed by the environment through which delivery occurs, the environment of the target for delivery, and/or the target itself. Biologically and chemically active agents are particularly vulnerable to such barriers.

In the delivery to animals of biologically active and chemically active pharmacological and therapeutic agents, barriers are also imposed by the body. Examples of physical barriers are the skin, lipid bi-layers and various organ membranes that are relatively impermeable to certain active agents but must be traversed before reaching a target, such as the circulatory system. Chemical barriers include, but are not limited to, pH variations in the gastrointestinal (GI) tract and degrading enzymes.

These barriers are of particular significance in the design of oral delivery systems. Oral delivery of many biologically or chemically active agents would be the route of choice for administration to animals if not for biological, chemical, and physical barriers. Among the numerous agents which are not typically amenable to oral administration are biologically or chemically active peptides, such as calcitonin and insulin; polysaccharides, and in particular mucopolysaccharides including, but not limited to, heparin; heparinoids; antibiotics; and other organic substances. These agents may be rapidly rendered ineffective or destroyed in the gastrointestinal tract by acid hydrolysis, enzymes, and the like. In addition, the size and structure of macromolecular drugs may prohibit absorption.

Earlier methods for orally administering vulnerable pharmacological agents have relied on the co-administration of adjuvants (*e.g.*, resorcinols and non-ionic surfactants such as polyoxyethylene oleyl ether and n-hexadecylpolyethylene ether) to increase artificially the permeability of the intestinal walls, as well as the co-administration of enzymatic inhibitors (*e.g*., pancreatic trypsin inhibitors, diisopropylfluorophosphate (DFF) and trasylol) to inhibit enzymatic degradation. Liposomes have also been described as drug delivery systems for insulin and heparin. However, broad spectrum use of such drug delivery systems is precluded because: (1) the systems require toxic amounts of adjuvants or inhibitors; (2) suitable low molecular weight cargos, i.e. active agents, are not available; (3) the systems exhibit poor stability and inadequate shelf life; (4) the systems are difficult to manufacture; (5) the systems fail to protect the active agent (cargo); (6) the systems adversely alter the active agent; or (7) the systems fail to allow or promote absorption of the active agent.

Proteinoid microspheres have been used to deliver pharmaceuticals. See, for example, U.S. Patent Nos. 5,401,516; 5,443,841; and Re. 35,862. In addition, certain modified amino acids have been used to deliver pharmaceuticals. See, for example, U.S. Patent Nos. 5,629,020; 5,643,957; 5,766,633; 5,776,888; and 5,866,536.

A polymer has been conjugated to a modified amino acid or a derivative thereof via a linkage group to provide for polymeric delivery agents. The modified polymer may be any polymer, but preferred polymers include, but are not limited to, polyethylene glycol (PEG), and derivatives thereof. See, for example, International Patent Publication No. WO 00/40203.

International Patent Publication Nos. WO 01/32130 and WO 01/32596 disclose particular phenyl amine carboxylic acid compounds and phenoxy carboxylic acid compounds for delivering active agents. International Publication No. WO 00/50386 also discloses amine delivery agents.

International Application No. PCT/US02/36552, filed November 13, 2002, published as International Application No. WO 03/045306, disclose phenoxy amine compounds and compositions for delivering active agents. Each of the above applications are hereby incorporated by reference.

WO 01/51454 relates to compounds for delivering active agents. The delivery agent is a compound having an amide group.

However, there is still a need for simple, inexpensive delivery systems which are easily prepared and which can deliver a broad range of active agents by various routes.

### SUMMARY OF THE INVENTION

The problem of the present invention is solved on the basis of claims 1 to 11.

The present invention provides a pharmaceutical composition comprising:
(A) a biologically active agent selected from proteins, peptides, polypeptides and any combination thereof; and
(B) at least one delivery agent compound having the formula 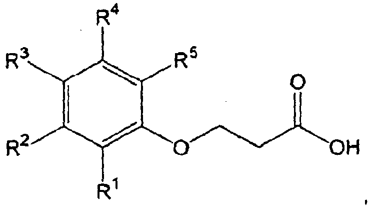
or a phannaceutically acceptable salt thereof, or a polyamino acid or peptide of which the delivery agent compound forms one or more unit thereof;
wherein R¹, R², R³, R⁴ and R⁵ are independently selected from H, halogen, and unsubstituted or substituted alkyl.

Delivery agent compounds of the present invention include those set forth below, including pharmaceutically acceptable salts thereof:

**Table 1:**

| **Compound No.** | **Structure** |
|---|---|
| 1 | 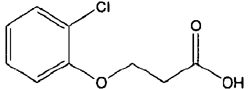 |
| 2 | 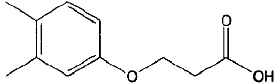 |
| 3 | 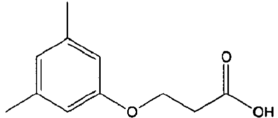 |
| 4 | 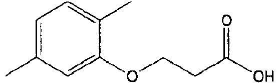 |
| 5 | 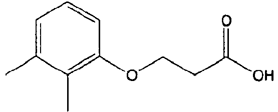 |
| 6 | 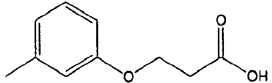 |
| 7 | 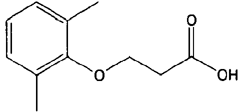 |
| 8 | 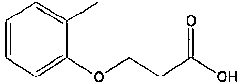 |
| 9 | 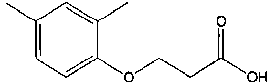 |

### DETAILED DESCRIPTION OF THE INVENTION

### Definitions

The term "delivery agent" as used herein refers to the propyl phenoxy ether compounds of the present invention, including crystalline polymorphic forms, amorphic forms, hydrates, and anhydrous forms thereof.

An "effective amount of drug" is an amount of the active agent (e.g., heparin) which is effective to treat or prevent a condition in a living organism to whom it is administered over some period of time, e.g., provides a therapeutic effect during a desired dosing interval. Effective doses will vary, as recognized by those skilled in the art, depending on the route of administration, excipient usage, and the possibility of co-usage with other agents for treating a condition.

The term "treat", "treating", or "treated" refers to administering an active agent with the purpose to cure, heal, alleviate, prevent, relieve, alter, remedy, ameliorate, improve, or affect a condition (e.g., a disease), the symptoms of the condition, or the predisposition toward the condition.

An "effective amount of delivery agent" is an amount of the delivery agent which promotes the absorption of a desired amount of the active agent via any route of administration (such as those discussed in this application including, but not limited to, the oral, nasal, pulmonary, dermal, vaginal, rectal and/or ocular route.

As used herein, the term "about" means within 10% of a given value, preferably within 5%, and more preferably within 1% of a given value. Alternatively, the term "about" means that a value can fall within a scientifically acceptable error range for that type of value, which will depend on how qualitative a measurement can be given the available tools.

### Delivery Agent Compounds

Delivery agent compounds of the present invention are selected from: 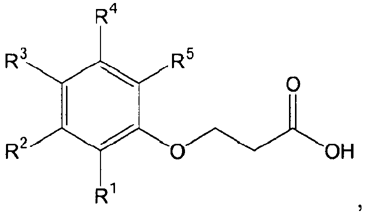 or a pharmaceutically acceptable salt thereof wherein R¹, R², R³, R⁴ and R⁵ are independently selected from H, halogen, unsubstituted or substituted alkyl .

In one embodiment, R¹, R², R³, R⁴ and R⁵ are independently selected from hydrogen, methyl, and chlorine groups.

The delivery agent compounds of the present invention may be in the form of the free base or phamaceutically acceptable pharmaceutically acceptable salts thereof. Suitable salts include, but are not limited to, organic and inorganic salts, for example ammonium, acetate salt, citrate salt, halide (preferably hydrochloride), hydroxide, sodium sulfate, nitrate, phosphate, alkoxy, perchlorate, tetrafluoroborate, carboxylate, mesylate, fumerate, malonate, succinate, tartrate, acetate, gluconate, and maleate. Preferred salts include, but are not limited to, sodium, sodium citrate and mesylate salts. The salts may also be solvates, including ethanol solvates, and hydrates. The delivery agent compound may be a multi-valent salt, such as a disodium salt.

Salts of the delivery agent compounds of the present invention may be prepared by methods known in the art. For example, citrate salts and mesylate salts may be prepared in ethanol, toluene and citric acid.

The delivery agent compound may be purified by recrystallization or by fractionation on one or more solid chromatographic supports, alone or linked in tandem. Suitable recrystallization solvent systems include, but are not limited to, ethanol, water, heptane, ethyl acetate, acetonitrile, acetone, methanol, and tetrahydrofuran (THF) and mixtures thereof. Fractionation may be performed on a suitable chromatographic support such as alumina, using methanol/n-propanol mixtures as the mobile phase; reverse phase chromatography using trifluoroacetic acid/acetonitrile mixtures as the mobile phase; and ion exchange chromatography using water or an appropriate buffer as the mobile phase. When anion exchange chromatography is performed, preferably a 0-500 mM sodium chloride gradient is employed.

### Active Agents

Active agents suitable for use in the present invention include biologically active agents including pesticides, pharmacological agents, and therapeutic agents. Suitable active agents include those that are rendered less effective, ineffective or are destroyed in the gastro-intestinal tract by acid hydrolysis, enzymes and the like. Also included as suitable active agents are those macromolecular agents whose physiochemical characteristics, such as, size, structure or charge, prohibit or impede absorption when dosed orally.

For example, biologically active agents suitable for use in the present invention include proteins; polypeptides; peptides; or any combination thereof.

Further examples include, the following, including synthetic, natural or recombinant sources thereof: calcitonin, including salmon, eel, porcine and human calcitonin; cholecystokinin (CCK) and CCK agonists including CCK-8; glucagon; glucagon-like peptide 1 (GLP-1), glucagon, and glucagon-like peptide 2 (GLP-2); insulin, including porcine, bovine, human, and human recombinant, optionally having counter ions including zinc, sodium, calcium and ammonium; insulin-like growth factor, including IGF-1; interferons, including α (e.g., interferon alfacon-1 (available as Infergen^{®} from InterMune, Inc. of Brisbane, CA)), alpha, β, omega and γ, interleukin-1; interleukin-2; interleukin-11; interleukin-21; leptin (OB protein); peptide YY (PYY) including PYY agonists, fragment 3-36; and polyethyleneglycol-modified derivatives of any of the above compounds.

### Delivery systems

The composition of the present invention comprises one or more delivery agent compounds of the present invention, and one or more active agents. In one embodiment, one or more of the delivery agent compounds, or salts of these compounds, or poly amino acids or peptides of which these compounds or salts form one or more of the units thereof, may be used as a delivery agent by mixing with the active agent prior to administration to form an administration composition.

The administration compositions may be in the form of a liquid The solution medium may be water (for example, for salmon calcitonin, parathyroid hormone, and erythropoietin). 25% aqueous propylene glycol (for example, for heparin) and phosphate buffer (for example, for rhGH). Other dosing vehicles include polyethylene glycol. Dosing solutions may be prepared by mixing a solution of the delivery agent compound with a solution of the active agent, just prior to administration. Alternatively, a solution of the delivery agent compound (or active agent) may be mixed with the solid form of the active agent (or delivery agent compound). The delivery agent compound and the active agent may also be mixed as dry powders. The delivery agent compound and the active agent can also be admixed during the manufacturing process.

The dosing solutions may optionally contain additives such as phosphate buffer salts, citric acid, glycols, or other dispersing agents. Stabilizing additives may be incorporated into the solution, preferably at a concentration ranging between about 0.1 and 20% (w/v).

The administration compositions may alternatively be in the form of a solid, such as a tablet, capsule or particle, such as a powder or sachet. Solid dosage forms may be prepared by mixing the solid form of the compound with the solid form of the active agent. Alternatively, a solid may be obtained from a solution of compound and active agent by methods known in the art, such as freeze-drying (lyophilization), precipitation, crystallization and solid dispersion.

The administration compositions of the present invention may also include one or more enzyme inhibitors. Such enzyme inhibitors include compounds such as actinonin or epiactinonin and derivatives thereof. Other enzyme inhibitors include aprotinin (Trasylol) and Bowman-Birk inhibitor.

The amount of active agent used in an administration composition of the present invention is an amount effective to accomplish the purpose of the particular active agent for the target indication. The amount of active agent in the compositions typically is a pharmacologically, biologically, therapeutically, or chemically effective amount. However, the amount can be less than that amount when the composition is used in a dosage unit form because the dosage unit form may contain a plurality of delivery agent compound/active agent compositions or may contain a divided pharmacologically, biologically, therapeutically, or chemically effective amount. The total effective amount can then be administered in cumulative units containing, in total, an effective amount of the active agent.

The total amount of active agent to be used can be determined by methods known to those skilled in the art. However, because the compositions of the invention may deliver active agents more efficiently than compositions containing the active agent alone, lower amounts of biologically or chemically active agents than those used in prior dosage unit forms or delivery systems can be administered to the subject, while still achieving the same blood levels and/or therapeutic effects.

The presently disclosed delivery agent compounds facilitate the delivery of biologically and chemically active agents, particularly in oral, intranasal, sublingual, intraduodenal, subcutaneous, buccal, intracolonic, rectal, vaginal, mucosal, pulmonary, transdermal, intradermal, parenteral, intravenous, intramuscular and ocular systems, as well as traversing the blood-brain barrier.

Dosage unit forms can also include any one or combination of excipients, diluents, disintegrants, lubricants, plasticizers, colorants, flavorants, taste-masking agents, sugars, sweeteners, salts, and dosing vehicles, including water, 1,2-propane diol, ethanol, olive oil, or any combination thereof.

The compounds and compositions of the subject invention are useful for administering biologically or chemically active agents to any animals, including but not limited to birds such as chickens; mammals, such as rodents, cows, pigs, dogs, cats, insects and primates, particularly humans.

The system is particularly advantageous for delivering chemically or biologically active agents that would otherwise be destroyed or rendered less effective by conditions encountered before the active agent reaches its target zone (i.e. the area in which the active agent of the delivery composition is to be released) and within the body of the animal to which they are administered. Particularly, the compounds and compositions of the present invention are useful for orally administering active agents, especially those that are not ordinarily orally deliverable, or those for which improved delivery is desired.

The compositions comprising the compounds and active agents have utility in the delivery of active agents to selected biological systems and in an increased or improved bioavailability of the active agent compared to administration of the active agent without the delivery agent. Delivery can be improved by delivering more active agent over a period of time, or in delivering the active agent in a particular time period (such as to effect quicker or delayed delivery), or in delivering the active agent at a specific time, or over a period of time (such as sustained delivery).

Another embodiment of the present invention is the composition of the present invention for use in the treatment or prevention of a disease or for achieving a desired physiological effect, such as any one of the diseases or conditions listed in the table below, in an animal. Preferably, an effective amount of the composition for the treatment or prevention of the desired disease or for achieving the desired physiological effect is administered. Specific indications for active agents can be found in the The Physicians' Desk Reference (58th Ed., 2004, Medical Economics Company, Inc., Montvale, NJ), and Fauci, AS, et. al., Harrison's Principles of Internal Medicine (14th Ed., 1998, McGraw-Hill Health Professions Division, New York. The active agents in the table below include their analogs, fragments, mimetics, and polyethylene glycol-modified derivatives (e.g., the PEGylated derivative of granulocyte colony stimulating factor sold as Neulasta®).

| **Active Agent** | **Disease and Physiological Effect** |
|---|---|
| Interferons, including α, β and γ | Viral infection, including chronic cancer, hepatitis, and multiple sclerosis |
| Interleukins (e.g. Interleukin-1; interleukin-2) | Viral infection; cancer; cell mediated immunity; and transplant rejection; |
| Insulin; Insulin-like growth factor IGF-1 | Diabetes |
| Immune Globulins, such as IVIg | smallpox, rabies, and diphtheria, Alzheimer's Disease; Primary immunodeficiencies; Acute Guillain-Barré syndrome; Chronic idiopathic demyelinating polyneuropathy (CIDP); Myasthenia gravis, polymyositis, and dermatomyositis; neonatal immune thrombocytopenia, heparin-induced thrombocytopenia, and antiphospholipid antibody syndrome: Posttransfusion purpura. |
| calcitonin; salmon calcitonin | Osteoporosis; diseases of the bone; bone pain; analgesic (including pain associated with osteoporosis or cancer) |
| Antigens | Infection |
| glucagon | improving glycemic control (e.g. treating hypoglycemia and controlling hypoglycemic reactions), obesity; a diagnostic aid in the radiogical examination of the stomach, duodenum, small bowel and colon; Treat acute poisoning With Cardiovascular Agents including, calcium channel blockers, beta blockers |
| GLP-1, Exendin - 3, Exendin - 4, Obestatin; MCHR1 receptor antagonists; selective inhibitor of 11-beta hydroxysteroid dehydrogenase type 1 | Diabetes; improving glycemic control (e.g. treating hypoglycemia and controlling hypoglycemic reactions), obesity |
| Peptide YY (PYY) and PYY-like Peptides (e.g. PYY[3-36]) | Obesity, Diabetes, Eating Disorders, Insulin-Resistance Syndromes |

For example, one embodiment of the present invention is insulin and at least one of the delivery agent compounds of the present invention for use in treating a patient having or susceptible to diabetes. Other active agents, including those set forth by way of non-limiting example in the above table, can be used in conjunction with the delivery agents of the present invention.

Following administration, the active agent present in the composition or dosage unit form is taken up into the circulation. The bioavailability of the agent can be readily assessed by measuring a known pharmacological activity in blood, e.g. an increase in blood clotting time caused by heparin, or a decrease in circulating calcium levels caused by calcitonin. Alternatively, the circulating levels of the active agent itself can be measured directly.

### Pharmaceutical Compositions

The pharmaceutical composition is preferably in solid form and may be formed into a solid dosage form. The solid dosage form can be a capsule, tablet or particle, such as a powder or sachet. The powder may be in the form of a sachet that is mixed with a liquid and administered. The solid dosage form may also be a topical delivery system, such as an ointment, cream or semi-solid. The solid dosage form contemplated may include a sustained release or controlled release system. Preferably, the solid dosage form is for oral administration.

The powder may be packed into capsules, or pressed into tablets, used in powder form, or incorporated into an ointment, cream or semi-solid. Methods for forming solid dosage forms are well known in the art.

The amount of delivery agent in the solid dosage form is a delivery effective amount and can be determined for any particular compound or biologically or chemically active agent by methods known to those skilled in the art. In one embodiment, the weight ratio of delivery agent:active ranges from about 1:5 or 5:1 to about 300:1 or 1:300. More specifically, the ratio of delivery agent:active may range from about 10:1 to about 200:1, or 50:1 to about 150:1. The amount of delivery agent used will vary according to the active agent, and the particular indication for which the active agent is administered.

For embodiments in which the active agent is ibandronate, the ratio of delivery agent:ibandronate may range from about 5:1 to about 300:1, or from about 10:1 to about 200:1, or 50:1 to about 150:1.

Following administration, the active agent in the dosage unit form is taken up into circulation. The bioavailability of the active agent is readily assessed by measuring a known pharmacological activity in blood, e.g. an increase in blood clotting time caused by heparin, or a decrease in circulating calcium levels caused by calcitonin. Alternatively, the circulating levels of the active agent itself can be measured directly.

The solid dosage form may include pharmaceutically acceptable additives, such as excipients, carriers, diluents, stabilizers, plasticizers, binders, glidants, disintegrants, bulking agents, lubricants, plasticizers, colorants, film formers, flavoring agents, preservatives, dosing vehicles, surfactants, and any combination of any of the foregoing. Preferably, these additives are pharmaceutically acceptable additives, such as those described in Remington's, The Science and Practice of Pharmacy, (Gennaro, A.R., ed., 19th edition, 1995, Mack Pub. Co.) .

Suitable binders include, starch, gelatine, sugars (such as sucrose, molasses and lactose), dibasic calcium phosphate dihydrate, natural and synthetic gums (such as acacia, sodium alginate, carboxymethyl cellulose, methyl cellulose, polyvinylpyrrolidone, polyethylene glycol, ethylcellulose, and waxes.

Suitable glidants include, talc, and silicon dioxide (silica) (e.g, fumed silica and colloidal silicon dioxide).

Suitable disintegrants include, starches, sodium starch glycolate, croscarmellose sodium, crospovidone, clays, celluloses (such as purified cellullose, methylcellulose, sodium carboxymethyl cellulose), alginates, pregelatinized corn starches, and gums (such as agar, guar, locust bean, karaya, pectin and tragacanth gums). A preferred disintegrant is sodium starch glycolate.

Suitable bulking agents include, starches (such as rice starch), microcrystalline cellulose, lactose (e.g., lactose monohydrate), sucrose, dextrose, mannitol, calcium sulfate, dicalcium sulfate, and tricalcium sulfate.

Suitable lubricants include, stearic acid, stearates (such as calcium stearate and magnesium stearate), talc, boric acid, sodium benzoate, sodium acetate, sodium fumarate, sodium chloride, polyethylene glycol, hydrogenated cottonseed, and castor oils.

Suitable surfactants include, sodium lauryl sulfate, hydroxylated soy lecithin, polysorbates, and block copolymers of propylene oxide and ethylene oxide.

### EXAMPLES

The following examples illustrate the invention. All parts are given by weight unless otherwise indicated.

Proton nuclear magnetic resonance (¹H NMR) analyses for the compounds listed below were conducted on a 300 MHz Bruker spectrometer using dimethyl sulfoxide (DMSO-d₆) as the solvent unless otherwise indicated.

The propyl phenoxy ether delivery agent compounds of the present invention may be prepared from the addition of phenol with acrylonitrile in the presence of t-BuOK (10% mol equiv.), followed by the hydrolysis in 37% HCl. The general scheme is shown below.

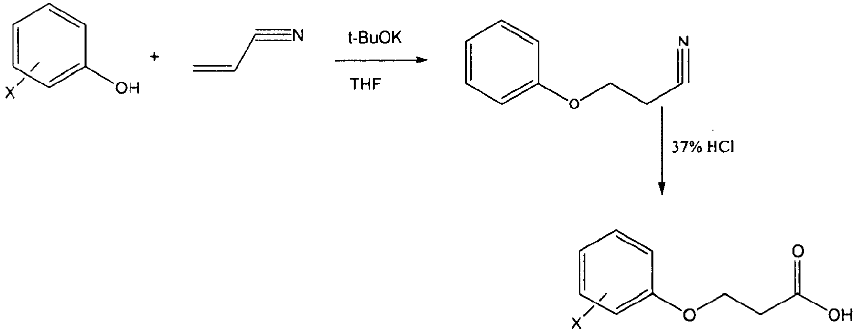

### Example 1: Preparation of Compounds

### Preparation of 3-2-Chloro-phenoxy-propionic acid (Compound 1):

At 25°C, to a stirred solution of *t*-BuOK in THF (1M, 6.0 ml, 6.0 mmol), 2-chlorophenol (7.72g, 60.0 mmol) was added, followed by the addition of acrylonitrile (20 ml). After the reaction mixture was heated to reflux for 6h, the excess of solvent was removed under vacuum. The aqueous NaOH (1N, 60 mL) was added to the reaction residue, which was then extracted with Et₂O (50 ml x 3). The organic phase was combined and washed with aqueous NaOH (1N, 10 ml x 3), and water (10 ml x 2) separately. The ether extract was dried with anhydrous sodium sulfate and then concentrated to give 3-(2-chloro-phenoxy)-propionitrile (6.3 g, 44.7 mmol) as a colorless oil, which was ready for hydrolysis. The mixture of 3-(2-chlorophenoxy)-propionitrile (6.3 g, 34.7 mmol) in concentrated hydrochloric acid (20 ml) was refluxed for 8h. Microanalysis Calc. for C₉H₉ClO₃ (200.62): C 53.88, H 4.52; found: C 53.81, H 4.58. ¹H-NMR (d₆-DMSO): 7.40 (dd, 1 arom. H); 7.29 (t-like, 1 arom. H); 7.16 (dd, 1 arom. H); 6.95 (td, 1 arom. H); 4.24 (t, -OC*H*₂-); 2.72 (t, -OCH₂C*H*₂-).

### Preparation of 3-(3,4-Dimethyl-phenoxy)-propionic acid (Compound 2):

At 25°C, to a stirred solution of t-BuOK in THF (1M, 6.0 ml, 6.0 mmol), 3,4-dimethylphenol (7.33 g, 60.0 mmol) was added, followed by the addition of acrylonitrile (20 ml). After the reaction mixture was heated to reflux for 6 h, the excess of solvent was removed under vacuum. The aqueous NaOH (1 N, 60ml) was added to the reaction residue, which was then extracted with Et₂O (50 ml x 3). The organic phase was combined and washed with aqueous NaOH (1 N, 10 ml x 3), water (10 ml x 2) separately. The ether extract was dried with anhydrous sodium sulfate and then concentrated to give 3-(3,4-dimethyl-phenoxy)-propionitrile (5.65 g, 32.2 mmol) as white solid, which was ready for hydrolysis.

The mixture of 3-(3,4-dimethyl-phenoxy)-propionitrile (5.65 g, 32.2 mmol) in concentrated hydrochloric acid (20 ml) was refluxed for 8 h. After cooling down, the resulting precipitate was collected by filtration and dissolved in aqueous NaOH (1 N, 40 ml). After the extraction with ether (20 ml), this aqueous solution was acidified with 6 N aqueous hydrochloric to generate white precipitate, which was collected by filtration to yield pure product as white powder (3.0 g, 25.7%). Mp 137-138°C. Micoanalysis Calc.for C11H14O3 (194.23): C 68.02, H 7.27; found: C 67.42, H 7.32. 1H-NMR (300 MHz, d6-DMSO): 7.01 (d, J(5,6) = 8.3, H-C(5)); 6.72 (d, J(2,6) = 2.5, H-C(2)); 6.63 (dd, J(5,6) = 8.3, J(2,6) = 2.5, H-C(6)); 4.10 (t, J = 6.1, - OCH2-); 2.65 (t, J = 6.1, -OCH2CH2-); 2.17, 2.13 (2s, 2 -CH3).

### Preparation of 3-(3,5-Dimethyl-phenoxy)-propionic acid (Compound 3):

The reaction of 3,5-dimethylphenol (7.33 g, 60.0 mmol) and acrylonitrile (20 ml) in the presence of t-BuOK in THF (1 M, 6.0 ml, 6.0 mmol) was performed as described for Compound 1 to give 3-(3,5-dimethyl-phenoxy)-propionitrile (6.24 g) as solid, which was ready for hydrolysis.

The hydrolysis of 3-(3,5-dimethyl-phenoxy)-propionitrile (6.24 g) was carried out as described for Compound 2 to yield pure product as white powder (2.08 g, 17.8%).1H-NMR (300 MHz, d6-DMSO): 6.57 (s, 1 arom. H); 6.53 (s, 2 arom. H); 4.11 (t, J = 6.1, -OCH2-); 2.66 (t, J = 6.1, -OCH2CH2-); 2.22 (s, 2 -CH3).

### Preparation of 3-(2,5-Dimethyl-phenoxy)-propionic acid (Compound 4):

The reaction of 2,5-dimethylphenol (7.33 g, 60.0 mmol) and acrylonitrile (20 ml) in the presence of t-BuOK in THY (1 M, 6.0 ml, 6.0 mmol) was performed as described for Compound 1 to give 3-(2,5-dimethyl-phenoxy)-propionitrile (6.60 g, 37.7 mmol) as oil, which was ready for hydrolysis.

The hydrolysis of 3-(2,5-dimethyl-phenoxy)-propionitrile (6.60 g, 37.7 mmol) was carried out as described for Compound 2 to yield pure product as white powder (6.1 g, 52.3%). Mp 94-95°. Micoanalysis Calc.for C11H14O3 (194.23): C 68.02, H 7.27; found: C 67.69, H 7.56. 1H-NMR (300 MHz, d6-DMSO): 6.98, 6.54 (AB, JAB = 8.3, H-C(3) and H-C(4)); 6.75 (s, H-C(6)); 4.14 (t, J = 6.0, -OCH2-); 2.68 (t, J = 6.0, -OCH2CH2-); 2.25, 2.04 (2s, 2 -CH3).

### Preparation of 3-(2,3-Dimethyl-phenoxy)-propionic acid (Compound 5):

The reaction of 2,3-dimethylphenol (7.33 g, 60.0 mmol) and acrylonitrile (20 ml) in the presence of t-BuOK in THF (1 M, 6.0 ml, 6.0 mmol) was performed as described for compound 1 to give 3-(2,3-dimethyl-phenoxy)-propionitrile (6.00 g) as oil, which was ready for hydrolysis.

The hydrolysis of 3-(2,3-dimethyl-phenoxy)-propionitrile (6.00 g) was carried out as described for compound 2 to yield pure product as white powder (4.8 g, 41.2%). 1H-NMR (400 MHz, D2O): 6.89 (m, 1 arom. H); 6.58 (m, 2 arom. H); 4.00 ((t, J = 6.4, -OCH2-); 2.52 (t, J = 6.4, -OCH2CH2-); 2.06, 1.89 (2s, 2 -CH3). 13C-NMR (100 MHz, D2O): 172.35(-C=O); 156.17; 137.22; 125.88; 124.27; 122.16; 109.42; 64.06; 34.20; 19.67; 11.27.

### Preparation of 3-m-Tolyloxy-propionic acid (Compound 6):

The reaction of 3-methylphenol (6.48 g, 60.0 mmol) and acrylonitrile (20 ml) in the presence of t-BuOK in THF (1 M, 6.0 ml, 6.0 mmol) was performed as described for Compound 1 to give 3-(3-methyl-phenoxy)-propionitrile (6.40 g) as solid, which was ready for hydrolysis.

The hydrolysis of 3-(3-methyl-phenoxy)-propionitrile (6.40 g) was carried out as described for Compound 2 to yield pure product as white powder 5.94 g, 54.9%) 1 H-NMR (400 MHz, D2O): 7.00 (m, l arm. H); 6.58 (m, 3 arom. H); 4.00 ((t, J = 6.4, -OCH2-); 2.55 (t, J = 6.4, -OCH2CH2-); 2.13 (s, CH3). 13C-NMR (100 MHz, D2O): 172.23(-C=O); 158.31; 138.96; 129.19; 121.35; 115.02; 111.38; 63.34; 34.20; 21.06.

### Preparation of 3-(2,6-Dimethyl-phenoxy)-propionic acid (Compound 7):

The reaction of 2,6-dimethylphenol (7.33 g, 60.0 mmol) and acrylonitrile (20 ml) in the presence of t-BuOK in THF (1 M, 6.0 ml, 6.0 mmol) was performed as described for Compound 1 to give 3-(2,6-dimethyl-phenoxy)-propionitrile (4.00 g, 22.8 mmol) as oil, which was ready for hydrolysis.

The hydrolysis of 3-(2,6-dimethyl-phenoxy)-propionitrile (4.00 g, 22.8 mmol) was carried out as described for Compound 2 to yield 3-(2,6-dimethyl-phenoxy)-propionic acid as oil (2.77 g, 14.3 mmol), which was then treated with of 1 M sodium trimethylsilanolate (13.0 ml, 13.0 mmol) to give sodium 3-(2,6-dimethyl-phenoxy)-propionate as white powder (2.47 g, 19.0%). 1H-NMR (400 MHz, D2O): 6.98 (m, 2 arom. H); 6.90 (m, 1 arom. H); 3.95 (t, J = 6.4, - OCH2-); 2.53 (t, J = 6.4, -OCH2CH2-); 2.14 (s, 2 -CH3). 13C-NMR (100 MHz, D2O): 179.96 (- C=O); 154.68; 131.63 (2 arom. C); 128.96 (2 arom. C); 124.66; 69.82; 38.29; 15.40 (2 -CH3).

### Preparation of 3-o-Tolyloxy-propionic acid (Compound 8):

The reaction of 2-methylphenol (6.48 g, 60.0 mmol) and acrylonitrile (20 ml) in the presence of t-BuOK in THF (1 M, 6.0 ml, 6.0 mmol) was performed as described for Compound 1 to give 3-(2-methyl-phenoxy)-propionitrile (6.50 g) as solid, which was ready for hydrolysis.

The hydrolysis of 3-(2-methyl-phenoxy)-propionitrile (6.50 g) was carried out as described for Compound 2 to yield pure product as white powder 5.62 g, 52.0%) 1H-NMR (400 MHz, D₂O): 7.12 (m, 2 arom. H); 6.93 (m, 1 arom. H); 6.83 (m, 1 arom. H); 4.14 ((t, J = 6.4, - OCH2-); 2.67 (t, J = 6.4, -OCH2CH2-); 2.12 (s, CH3). 13C-NMR (100 MHz, D2O): 172.45(-C=O); 156.55; 130.47; 127.05; 125.91; 120.45; 111.53; 63.89; 34.42; 15.89.

### Preparation of 3-(2,4-Dimethyl-phenoxy)-propionic acid (Compound 9):

The reaction of 2,4-dimethylphenol (5.44 g, 44.5 mmol) and acrylonitrile (15 ml) in the presence of t-BuOK in THF (1 M, 4.5 ml, 4.5 mmol) was performed as described for Compound 1 to give 3-(2,4-dimethyl-phenoxy)-propionitrile (4.40 g, 25.1 mmol) as solid, which was ready for hydrolysis.

The hydrolysis of 3-(2,4-dimethyl-phenoxy)-propionitrile (4.40 g, 25.1 mmol) was carried out as described for Compound 2 to yield pure product as white powder (2.98 g, 34.5%). 1H-NMR (400 MHz, d6-DMSO): 6.80 (m, 2 arom. H); 6.68 (m, 1 arom. H); 3.97 (t, J = 6.4. -OCH2-); 2.55 (t, J = 6.4, -OCH2CH2-); 2.07, 1.95 (2s, 2 -CH3). 13C-NMR (100 MHz, d6-DMSO): 172.35 (-C=O); 154.33; 131.09; 128.91; 127.03; 125.58; 111.51; 63.93; 34.20; 20.06; 15.71.

### Example 2: In Vivo Delivery of Insulin to Fasted Rats via Oral Gavage

Insulin (human recombinant) was obtained from ICN Biomedicals (Aurora, OH) as a bulk powder. To prepare stock solutions, insulin was dissolved in deionized water (pH∼6.5) to obtain a concentration of 15 mg/ml. Stock solutions were kept frozen at -20°C in 1.0-ml aliquots until used. For dosing solutions, delivery agent was dissolved in deionized water to obtain a final concentration of 200 mg/ml. The free acid form of delivery agent was converted to the sodium salt by adding one equivalent of sodium hydroxide. Solutions were vortexed, sonicated, and heated, and if necessary, additional sodium hydroxide was added in µl quantities to achieve uniform solubility. Solutions were adjusted to a pH of 3.5-8.5 by the addition of either hydrochloric acid or sodium hydroxide. Insulin stock (typically 66.7 µls) was then added to the delivery agent solution to obtain a final concentration of 0.5 mg/ml. After solubilization and drug addition, solutions were brought to final volume by the addition of deionized water.

Insulin was administered to male, Sprague-Dawley rats either alone or in combination with an Emisphere delivery agent by oral gavage (PO). Rats were fasted for 18-24 hours prior to dosing. For dosing, a Rusch 8 French catheter was cut to 11 cm in length and adapted to fit a 1-ml syringe. The syringe was filled with dosing solution and the catheter was wiped dry of excess solution. The catheter was inserted into the rat mouth and fed down the esophagus (10.0 cm). The dosing solution was delivered by pressing the syringe plunger while holding the rat in an upright position.

### Sample collection and handling: Insulin

During blood sampling, rats were exposed briefly (∼10 sec) to carbon dioxide until prostrate, immediately prior to each sampling time point. For blood sampling, a 77-mm capillary tube was inserted into the retroorbital sinus. Typically, blood samples were collected prior to dosing (time 0) and at 15, 30, 45, and 60 minutes after dosing. Samples were collected into capiject® tubes (Terumo Corporation, Tokyo, Japan) containing a clot activator (red top, serum separator tubes). Samples were allowed to clot for ∼20 min at 4°C. After clotting, samples were centrifuged at 10,000 rpm for 4 minutes at 6°C in order to separate the serum. Serum was collected into eppendorf tubes and frozen at -20°C until assayed.

### Sample collection and handling: Whole blood glucose

In order to determine the pharmacodynamic response, a hand-held glucometer (OneTouch Ultra, LifeScan® (Johnson & Johnson, New Brunswick, New Jersey)) was used to measure whole blood glucose after administration of insulin or insulin and delivery agent. Samples were collected either from the retroorbital sinus (see Sample collection and handling: Insulin) or from the tail artery (i.e. tail clip). For tail clipping, the tip of the tail was severed approximately 5 mm from the tip using a scalpel blade. After discarding the first drop of blood, a small sample (∼5-10 µl) was touched to the glucometer test strip (OneTouch Ultra, LifeScan) and a blood glucose reading was generated by the meter. For each subsequent sampling time point, the clot formed at the tip of the tail was broken up and a fresh sample was collected. Typically, samples were collected prior to dosing (time 0) and at 15, 30, 45, and 60 minutes after dosing.

### Bioanalytical method and data analysis- insulin assay

Concentrations of insulin were quantified in rat serum using a sandwich-type ELISA (kit; Diagnostic Systems Laboratories, Inc., Webster, TX). The calibrated assay range was 12.5-250.0 µlU/mL. Serum from rats was obtained internally from stock animals and used to prepare calibration standards and low and high quality control samples (LQC, HQC). The low and high quality control samples for the second curve were prepared at 30 and 150 µlU/mL, respectively. Calibration standards were prepared fresh daily and quality control samples were stored at a nominal temperature of -20°C. Concentration values (test samples) were read from the standard curve, averaged for each time point (n=5), and plotted as mean serum concentration of insulin (± SEM) versus time.

| Delivery Agent Compound No. | Delivery Agent Dose | Insulin Dose | % Glucose Cₘᵢₙ(Rat) |
|---|---|---|---|
| 1 | 200mg/kg | 0.5 mg/kg | -47.0 |
| 2 | 200mg/kg | 0.5 mg/kg | -48.2 |
| 3 | 200mg/kg | 0.5 mg/kg | -16.6 |
| 4 | 200mg/kg | 0.5 mg/kg | -39.6 |
| 5 | 200mg/kg | 0.5 mg/kg | -22.2 |
| 6 | 200mg/kg | 0.5 mg/kg | -29.3 |
| 7 | 200mg/kg | 0.5 mg/kg | -42.1 |
| 8 | 200mg/kg | 0.5 mg/kg | -24.2 |
| 9 | 200mg/kg | 05 mg/kg | -40.3 |

## Claims

1. A pharmaceutical composition comprising:
(A) a biologically active agent selected from proteins, peptides, polypeptides, and any combination thereof; and
(B) at least one delivery agent compound having the formula 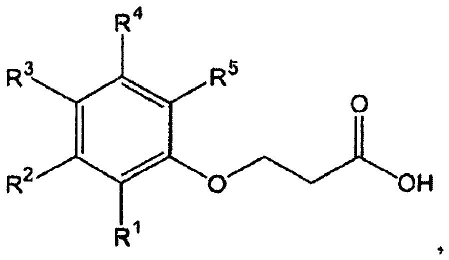 or a pharmaceutically acceptable salt thereof, or a polyamino acid or peptide of which the delivery agent compound forms one or more unit thereof;
wherein R¹, R², R³, R⁴ and R⁵ are independently selected from H, halogen, and unsubstituted or substituted alkyl.

2. The pharmaceutical composition of claim 1, wherein R¹, R², R³, R⁴ and R⁵ are independently selected from hydrogen, methyl and chlorine.

3. The pharmaceutical composition of claim 1 comprising:
(A) a biologically active agent selected from proteins, peptides, polypeptides, and any combination thereof; and
(B) at least one delivery agent compound selected from the group consisting of: 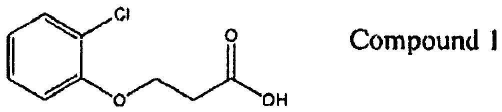 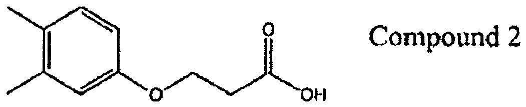 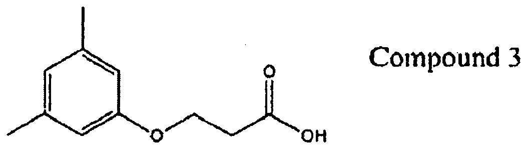 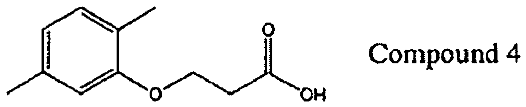 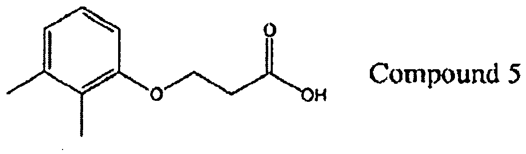 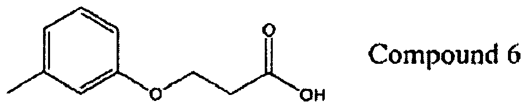 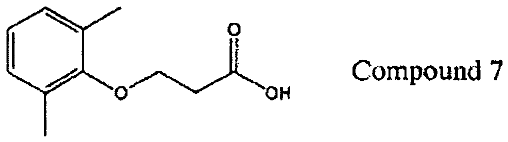 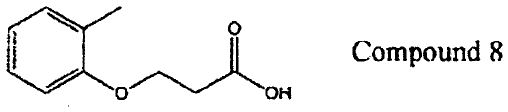 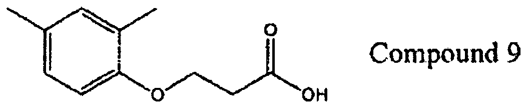 and pharmaceutically acceptable salts thereof.

4. The pharmaceutical composition of claim 1, wherein the biologically active agent is selected from the group consisting of: calcitonin gene-related proteins; calcitonin, salmon calcitonin, eel calcitonin, porcine calcitonin, human calcitonin; cholecystokinin (CCK) and CCK agonists, CCK-8; glucagon; glucagon-like peptide I (GLP-1), glucagon, glucagon-like peptide 2 (GLP-2); insulin, porcine insulin, bovine insulin, human insulin, human recombinant insulin, optionally having counter ions including zinc, sodium, calcium and ammonium; insulin-like growth factor, IGF-1; interferons, α-interferon, β-interferon, omega interferon, γ interferon; interleukin-1; interleukin-2; interleukin-11; interleukin-21; leptin (OB protein); peptide YY (PYY), PYY agonists, PYY₃₋₃₆; and polyethylene glycol-modified derivatives of any of the above compounds, and any combination thereof.

5. The pharmaceutical composition of claim 4, wherein the biologically active agent comprises insulin, calcitonin, or polyethylene glycol-modified derivatives of these compounds; or any combination thereof.

6. A dosage unit form comprising:
(A) a pharmaceutical composition of claim 1; and
(B)
(a) an excipient
(b) a diluent,
(c) a disintegrant,
(d) a lubricant,
(e) a plasticizer,
(f) a colorant,
(g) a dosing vehicle, or
(h) any combination thereof.

7. The dosage unit form of claim 6, wherein the biologically active agent is selected from the group consisting of calcitonin, salmon calcitonin, eel calcitonin, porcine calcitonin, human calcitonin; cholecystokinin (CCK) and CCK agonists, CCK-8; glucagon; glucagon-like peptide 1 (GLP-1), glucagon, glucagon-like peptide 2 (GLP-2); insulin, porcine insulin, bovine insulin, human insulin, human recombinant insulin, optionally having counter ions including zinc, sodium, calcium and ammonium; insulin-like growth factor, IGF-1; interferons, α-interferon, β-interferon, omega interferon, γ interferon; interleukin-1; interleukin-2; interleukin-11; interleukin-21; peptide YY (PYY), PYY agonists, PYY₃₋₃₆; and polyethylene glycol-modified derivatives of any of the above compounds, and any combination thereof.

8. The pharmaceutical composition of claim 1, wherein the biologically active agent comprises insulin, calcitonin, or polyethylene glycol-modified derivatives of these compounds; or any combination thereof.

9. The dosage unit form of claim 6, wherein the biologically active agent comprises insulin.

10. The pharmaceutical composition of claim 1, where the biologically active agent is insulin and the delivery agent is:

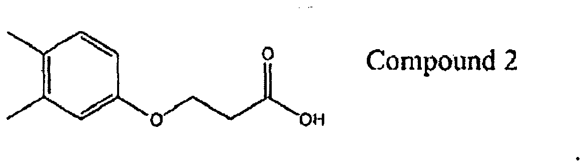

11. The dosage unit form of claim 6, where the biologically active agent is insulin and the delivery agent is:

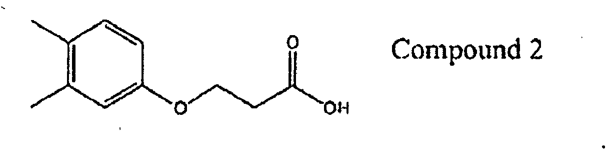

## Patentansprüche

1. Pharmazeutische Zusammensetzung umfassend:
(A) ein biologisch aktives Mittel ausgewählt aus Proteinen, Peptiden, Polypeptiden und jeder Kombination davon; und
(B) mindestens eine Verabreichungsmittelverbindung (delivery agent compound) mit der Formel, 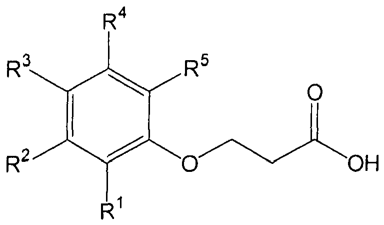 oder ein pharmazeutisch verträgliches Salz davon, oder eine Polyaminosäure oder ein Peptid, von welchem die Verabreichungsmittelverbindung eine oder mehrere Einheiten davon bildet;
wobei R¹, R², R³, R⁴ und R⁵ unabhängig voneinander ausgewählt sind aus H, Halogen und unsubstituiertes oder substituiertes Alkyl.

2. Pharmazeutische Zusammensetzung nach Anspruch 1, wobei R¹, R², R³, R⁴ und R⁵ unabhängig voneinander ausgewählt sind aus Wasserstoff, Methyl und Chlor.

3. Pharmazeutische Zusammensetzung nach Anspruch 1, umfassend:
(A) ein biologisch aktives Mittel ausgewählt aus Proteinen, Peptiden, Polypeptiden und jeder Kombination davon; und
(B) mindestens eine Verabreichungsmittelverbindung ausgewählt aus der Gruppe bestehend aus: 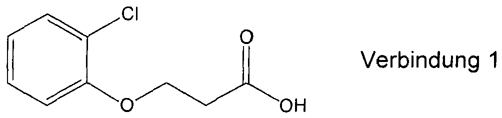 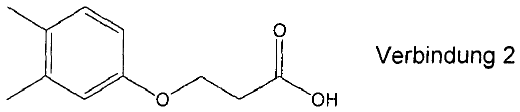 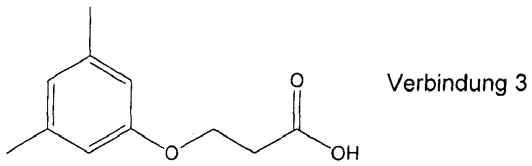 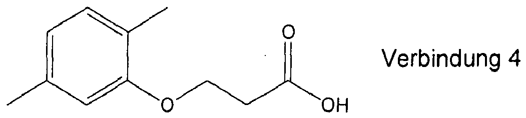 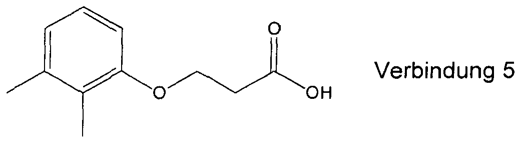 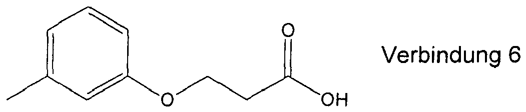 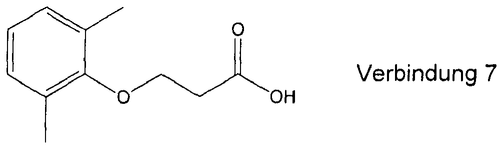 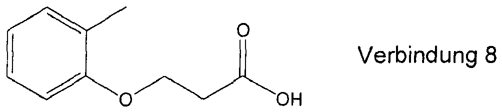 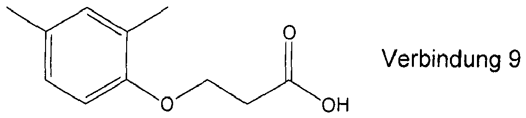 und pharmazeutisch verträgliche Salze davon.

4. Pharmazeutische Zusammensetzung nach Anpruch 1, wobei das biologisch aktive Mittel ausgewählt ist aus der Gruppe bestehend aus: Calcitonin Gene-Related Proteine; Calcitonin, Lachscalcitonin, Aalcalcitonin, Schweinecalcitonin, menschliches Calcitonin; Cholecystokinin (CCK) und CCK Agonisten, CCK-8; Glucagon; Glucagon-like Peptide 1 (GLP-1), Glucagon, Glucagon-like Peptide 2 (GLP-2); Insulin, Schweineinsulin, Rinderinsulin, menschliches Insulin, menschliches rekombinantes Insulin, optional mit Gegenionen einschließlich Zink, Natrium, Calcium und Ammonium; Insulin-ähnlicher Wachstumsfaktor, IGF-1; Interferone, α-Interferon, β-Interferon, omega-Interferon, γ-Interferon; Interleukin-1; Interleukin-2; Interleukin-11; Interleukin-21; Leptin (OB Protein); Peptid YY (PYY), PYY Agonisten, PYY₃₋₃₆; und Polyethylenglykol-modifizierte Derivate von einer der oben genannten Verbindungen und jede Kombination davon.

5. Pharmazeutische Zusammensetzung nach Anspruch 4, wobei das biologisch aktive Mittel Insulin, Calcitonin oder Polyethylenglykol-modifizierte Derivate von diesen Verbindungen umfasst; oder jede Kombination davon.

6. Dosierungseinheitsform umfassend:
(A) eine pharmazeutische Zusammensetzung nach Anspruch 1; und
(B)
(a) einen Trägerstoff,
(b) einen Verdünner,
(c) ein Disintegrierungsmittel,
(d) ein Schmiermittel,
(e) einen Weichmacher,
(f) einen Farbstoff,
(g) ein Dosierhilfsmittel, oder
(h) jede Kombination davon

7. Dosierungseinheitsform nach Anspruch 6, wobei das biologisch aktive Mittel ausgewählt ist aus der Gruppe bestehend aus Calcitonin, Lachscalcitonin, Aalcalcitonin, Schweinecalcitonin, menschliches Calcitonin; Cholecystokinin (CCK) und CCK Agonisten, CCK-8; Glucagon; Glucagon-like Peptide 1 (GLP-1), Glucagon, Glucagon-like Peptide 2 (GLP-2); Insulin, Schweineinsulin, Rinderinsulin, menschliches Insulin, menschliches rekombinantes Insulin, optional mit Gegenionen einschließlich Zink, Natrium, Calcium und Ammonium; Insulin-ähnlicher Wachstumsfaktor, IGF-1; Interferone, α-Interferon, β-Interferon, omega-Interferon, γ-Interferon; Interleukin-1; Interleukin-2; Interleukin-11; Interleukin-21; Peptid YY (PYY), PYY Agonisten, PYY₃₋₃₆; und Polyethylenglykol-modifizierte Derivate von einer der oben genannten Verbindungen und jede Kombination davon.

8. Pharmazeutische Zusammensetzung nach Anspruch 1, wobei das biologisch aktive Mittel Insulin, Calcitonin oder Polyethylenglykol-modifizierte Derivate von diesen Verbindungen umfasst; oder jede Kombination davon.

9. Dosierungseinheitsform nach Anspruch 6, wobei das biologisch aktive Mittel Insulin umfasst.

10. Pharmazeutische Zusammensetzung nach Anspruch 1, wobei das biologisch aktive Mittel Insulin ist und das Verabreichungsmittel 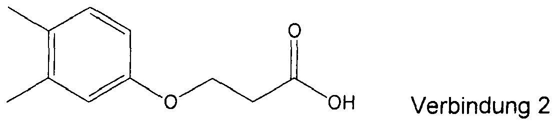 ist.

11. Dosierungseinheitsform nach Anspruch 6, wobei das biologisch aktive Mittel Insulin ist und das Verabreichungsmittel 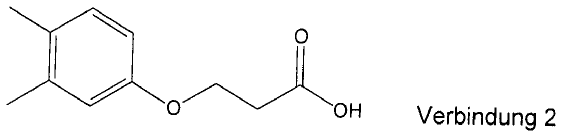 ist.

## Revendications

1. Une composition pharmaceutique comprenant:
(A) un agent biologiquement actif sélectionné parmi les protéines, les peptides, les polypeptides, et n'importe quelle combinaison de ceux-ci; et
(B) au moins un composé d'agent d'administration ayant la formule 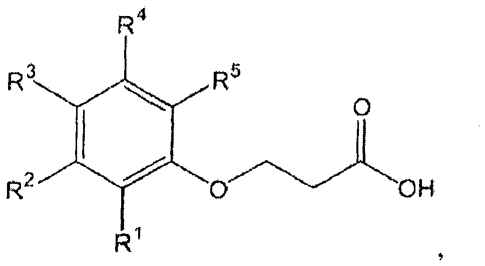 ou un sel pharmaceutiquement acceptable de celui-ci, ou un polyaminoacide ou un peptide, dont le composé d'agent d'administration forme une ou plusieurs unités de ceux-ci ;
dans laquelle R¹, R², R³, R⁴ et R⁵ sont indépendamment sélectionnés parmi H, un halogène, et un groupe alkyle non substitué ou substitué.

2. La composition pharmaceutique selon la revendication 1, dans laquelle R¹, R², R³, R⁴ et R⁵ sont indépendamment sélectionnés parmi un hydrogène, un méthyle et un chlore.

3. La composition pharmaceutique selon la revendication 1 comprenant:
(A) un agent biologiquement actif sélectionné parmi les protéines, les peptides, les polypeptides, et n'importe quelle combinaison de ceux-ci; et
(B) au moins un composé d'agent d'administration sélectionné parmi le groupe consistant en: 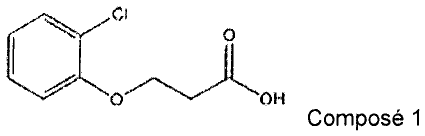 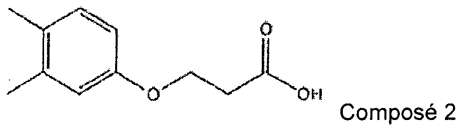 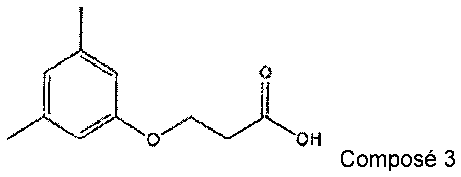 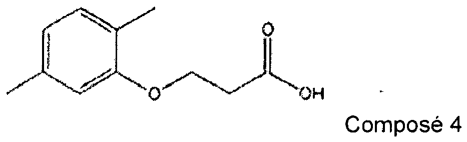 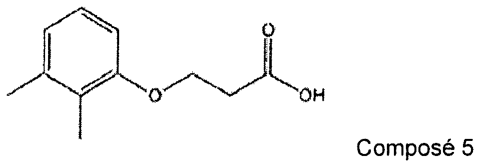 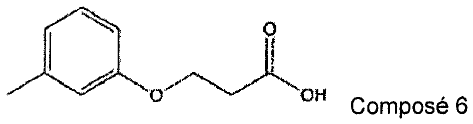 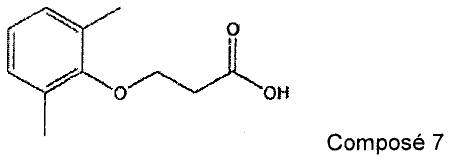 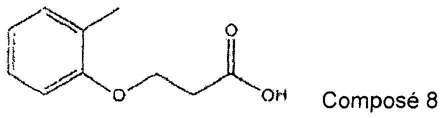 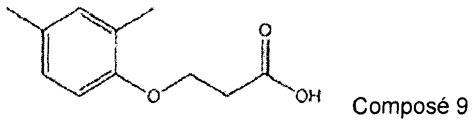 et des sels pharmaceutiquement acceptables de ceux-ci.

4. La composition pharmaceutique selon la revendication 1, dans laquelle l'agent biologiquement actif est sélectionné parmi le groupe consistant en: des protéines reliées à un gène calcitonine; une calcitonine, une calcitonine de saumon, une calcitonine d'anguille, une calcitonine porcine, une calcitonine humaine; une cholécystokinine (CCK) et des agonistes CCK, CCK-8; un glucagon; un peptide type glucagon 1 (GLP-1), un glucagon, un peptide type glucagon 2 (GLP-2); une insuline, une insuline porcine, une insuline bovine, une insuline humaine, une insuline recombinante humaine, ayant éventuellement des contre ions incluant le zinc, le sodium, le calcium et l'ammonium; un facteur de croissance type insuline, IGF-1; des interférons, un α-interféron, un β-interféron, un interféron oméga, un γ-interféron; une interleukine-1; une interleukine-2; une interleukine-11; une interleukine-21; une leptine (une protéine OB); un peptide YY (PYY), des agonistes PYY, PYY_{3-36;} et des dérivés modifiés de polyéthylène glycol de n'importe quels des composés ci-dessus et n'importe quelle combinaison de ceux-ci.

5. La composition pharmaceutique selon la revendication 4, dans laquelle l'agent biologiquement actif comprend une insuline, une calcitonine, ou des dérivés modifiés de polyéthylène glycol de ces composés; ou n'importe quelle combinaison de ceux-ci.

6. Une forme d'unité de dosage comprenant:
(A) une composition pharmaceutique selon la revendication 1; et
(B)
(a) un excipient,
(b) un diluent,
(c) un agent de désintégration,
(d) un lubrifiant,
(e) un plastifiant,
(f) un colorant,
(g) un véhicule de dosage, ou
(h) n'importe quelle combinaison de ceux-ci.

7. La forme d'unité de dosage selon la revendication 6, dans laquelle l'agent biologiquement actif est sélectionné parmi le groupe consistant en une calcitonine, une calcitonine de saumon, une calcitonine d'anguille, une calcitonine porcine, une calcitonine humaine; une cholécystokinine (CCK) et des agonistes CCK, CCK-8; un glucagon; un peptide type glucagon 1 (GLP-1), un glucagon, un peptide type glucagon 2 (GLP-2); une insuline, une insuline porcine, une insuline bovine, une insuline humaine, une insuline recombinante humaine, ayant éventuellement des contre ions incluant le zinc, le sodium, le calcium et l'ammonium; un facteur de croissance type insuline, IGF-1; des interférons, un α-interféron, un β-interféron, un interféron oméga, un γ-interféron; une interleukine-1; une interleukine-2; une interleukine-11; une interleukine-21; un peptide YY (PYY), des agonistes PYY, PYY₃₋₃₆; et des dérivés modifiés de polyéthylène glycol de n'importe quels des composés ci-dessus et n'importe quelle combinaison de ceux-ci.

8. La composition pharmaceutique selon la revendication 1, dans laquelle l'agent biologiquement actif comprend l'insuline, la calcitonine, ou des dérivés modifiés de polyéthylène glycol de ces composés; ou n'importe quelle combinaison de ceux-ci.

9. La forme d'unité de dosage selon la revendication 6, dans laquelle l'agent biologiquement actif comprend de l'insuline.

10. La composition pharmaceutique selon la revendication 1, dans laquelle l'agent biologiquement actif est de l'insuline et l'agent d'administration est:

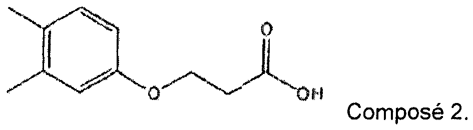

11. La forme d'unité de dosage selon la revendication 6, dans laquelle l'agent biologiquement actif est de l'insuline et l'agent d'administration est:
